# EUROPEAN PATENT APPLICATION

(11) **EP 4 458 845 A1**
(43) Date of publication of application: **06.11.2024**
(21) Application number: 22890195.5
(22) Date of filing: 12.10.2022
(51) Int. Cl.: C07K 14/47, C07K 14/485, A61K 38/00, C12N 15/62, C12N 15/63, A61P 1/00

(54) **RECOMBINANT PROTEIN FOR PREVENTING OR TREATING ULCERATIVE COLITIS, AND COMPOSITION FOR PREVENTING OR TREATING ULCERATIVE COLITIS COMPRISING SAME**

(30) Priority: 05.11.2021 KR 20210151300
(71) Applicant: NEXEL CO., LTD., Seoul 02580 (KR)
(72) Inventor: KIM, Changgeun, Seoul 07628 (KR); KIM, Gukdo, Seoul 06911 (KR); LEE, Jaehun, Seoul 05328 (KR); KIM, Minkyung, Seoul 07634 (KR); KIM, Joonchul, Seoul 06226 (KR); PARK, Junghyuck, Seongnam-si Seoul 13606 (KR); WOO, Donghun, Seoul 07900 (KR); HAN, Choongseong, Seoul 07959 (KR)
(74) Representative: dompatent von Kreisler Selting Werner - Partnerschaft von Patent- und Rechtsanwälten mbB
(86) International application number: PCT/KR2022/015344
(87) International publication number: WO 2023/080460

(57) **Abstract**

Provided is recombinant protein for ulcerative colitis treatment or prevention that is recombinant protein based on milk fat globule-EGF factor 8 (MFG-E8) protein, consisting of an amino acid sequence of SEQ ID NO: 1 or SEQ ID NO: 3, to be administered to a target for treatment or prevention, thereby expecting treatment or prevention of ulcerative colitis. Further, provided is a composition for ulcerative colitis treatment or prevention containing recombinant protein based on milk fat globule-EGF factor 8 (MFG-E8) protein, consisting of an amino acid sequence of SEQ ID NO: 1 or SEQ ID NO: 3, as an active ingredient.

## Description

### [Technical Field]

The present invention relates to recombinant protein for ulcerative colitis treatment or prevention, composition for ulcerative colitis treatment or prevention containing the same, and related various uses thereof.

### [Background Art]

Ulcerative colitis occurs when inflammation occurs in the large intestine and inflammatory cells invade a mucosa and a submucosa. The most common symptoms are bloody stool. Further, the symptoms may include diarrhea, mucous stool, abdominal pain, stool retention, tenesmus, and even constitutional symptoms such as weight loss, fever, loss of appetite, general weakness, nausea, and vomiting.

Normal large intestine tissues are divided into a mucosa, a submucosa, muscle layers, and a serosa. With the development of ulcerative colitis, pathological changes appear in the mucosa and submucosa. Currently, an exact cause thereof is not known, but it is a chronic recurrent disease for which the body's excessive immune response to bacteria normally present in the intestine is considered an important pathogenic factor, as well as environmental and genetic factors.

Such ulcerative colitis has traditionally had a high incidence in meat-eating regions such as the United States and Europe. For example, in the United States, about a million patients occur every year. Recently, the number of patients is increasing in Asian countries. In particular, in Korea, the number of hospitalized and outpatient patients with ulcerative colitis under the disease code K51 in 2020 has increased by about 1.7 times compared with 2010, and the cost of nursing care has also increased by more than 3 times compared with 2010.

In this regard, 5-aminosalicylic acid (5-ASA) and biological agents are used as standard drug treatments for ulcerative colitis, but 20% to 40% of patients undergo colectomy due to failed drug treatment or side effects.

To solve this problem, Korean Patent Registration NO. 10-2319078 (hereinafter, referred to as 'the prior art') has proposed a pharmaceutical composition for treating ulcerative colitis.

### [Disclosure]

### [Technical Problem]

The present invention has been made in view of the above problems, and it is an object of the present invention to provide recombinant protein for treating or preventing ulcerative colitis, capable of delaying onset of the ulcerative colitis or treating the disease, and a composition containing the same as an active ingredient.

### [Technical Solution]

In accordance with the present invention, the above and other objects can be accomplished by the provision of recombinant protein for ulcerative colitis treatment or prevention that is recombinant protein based on milk fat globule-EGF factor 8 (MFG-E8) protein, consisting of an amino acid sequence of SEQ ID NO: 1 or SEQ ID NO: 3.

Here, the recombinant protein may reduce, compared with a case in which the recombinant protein is not administered, degrees of crypt damage, an inflammation severity, and an inflammation extent in a mucosa of a large intestinal tissue that is a target for treatment or prevention.

Further, the recombinant protein may reduce, compared with a case in which the recombinant protein is not administered, degrees of erosion and epithelial hyperplasia in a mucosa of a large intestinal tissue that is a target for treatment or prevention.

In addition, the recombinant protein may reduce, compared with a case in which the recombinant protein is not administered, a concentration of interleukin-6 (IL-6) in blood of a target for treatment or prevention.

Further, the recombinant protein may reduce, compared with a case in which the recombinant protein is not administered, degrees of weight loss and colon length shrinkage caused by ulcerative colitis of a target for treatment or prevention.

In addition, the recombinant protein may be formulated for intralesional administration, intravascular administration, subcutaneous administration, intranasal administration, or intraperitoneal administration.

In accordance with another aspect of the present invention, there is provided a composition for ulcerative colitis treatment or prevention containing the recombinant protein that is recombinant protein based on milk fat globule-EGF factor 8 (MFG-E8) protein, consisting of an amino acid sequence of SEQ ID NO: 1 or SEQ ID NO: 3 as an active ingredient.

In accordance with still another aspect of the present invention, there is provided a gene encoding the recombinant protein that is recombinant protein based on milk fat globule-EGF factor 8 (MFG-E8) protein, consisting of an amino acid sequence of SEQ ID NO: 1 or SEQ ID NO: 3.

In accordance with a further another aspect of the present invention, there is provided a recombinant vector comprising a gene encoding the recombinant protein that is recombinant protein based on milk fat globule-EGF factor 8 (MFG-E8) protein, consisting of an amino acid sequence of SEQ ID NO: 1 or SEQ ID NO: 3.

### [Advantageous Effects]

According to the present invention, the following effects are achieved through administration of the recombinant protein for treatment or prevention of ulcerative colitis.

First, it is possible to treat or prevent ulcerative colitis of a target for treatment or prevention.

Second, it is possible to significantly lower the degrees of crypt damage, inflammation severity, and inflammation extent in a mucosa of a large intestinal tissue of the target for treatment or prevention.

Third, it is possible to significantly reduce the degrees of erosion and epithelial hyperplasia in the mucosa of the large intestinal tissue of the target for treatment or prevention.

Fourth, it is possible to significantly lower the concentration of interleukin-6 (IL-6, Interleukin 6) in blood of the target for treatment or prevention.

Fifth, it is possible to significantly reduce the degrees of weight loss and colon length shrinkage caused by ulcerative colitis of the target for treatment or prevention.

### [Description of Drawings]

The above and other objects, features and other advantages of the present invention will be more clearly understood from the following detailed description taken in conjunction with the accompanying drawings, in which:
FIG. 1 shows an amino acid sequence according to a first example of recombinant protein (NP-011) for treating or preventing ulcerative colitis according to the present invention;
FIG. 2 shows a DNA base sequence of a gene that may be inserted into a structure of backbone vector shown in FIG. 5 and coats the amino acid sequence according to the first example of the recombinant protein (NP-011) for treating or preventing the ulcerative colitis according to the present invention;
FIG. 3 shows an amino acid sequence according to a second example of the recombinant protein (NP-011) for treating or preventing the ulcerative colitis according to the present invention;
FIG. 4 shows a DNA base sequence of a gene that may be inserted into the structure of the backbone vector shown in FIG. 5 and coats the amino acid sequence according to the second example of the recombinant protein (NP-011) for treating or preventing the ulcerative colitis according to the present invention;
FIG. 5 shows the structure of the backbone vector in which the gene for coating the recombinant protein (NP-011) for treating or preventing the ulcerative colitis according to the present invention is inserted;
FIG. 6 and FIG. 10 are schematic diagrams showing a production process of an animal test model designed to verify effects of the recombinant protein (NP-011) for treating or preventing the ulcerative colitis according to the present invention;
FIG. 7 and FIG. 11 show results obtained by analyzing the degrees of crypt damage and inflammation severity in a mucosa of a large intestine tissue in animal test models designed to verify the effects of the recombinant protein (NP-011) for treating or preventing the ulcerative colitis according to the present invention;
FIG. 8 and FIG. 12 are graphs comparatively showing weight change patterns and differences in the animal test models designed to verify effects of the recombinant protein (NP-011) for treating or preventing ulcerative colitis according to the present invention;
FIG. 9 and FIG. 13 are graphs comparatively showing colon length changes and differences in the animal test models designed to verify the effects of the recombinant protein (NP-011) for treating or preventing ulcerative colitis according to the present invention; and
FIG. 14 is a graph comparatively showing change patterns and differences in an in-blood interleukin-6 (IL-6) concentration in the animal test models designed to verify the effects of the recombinant protein (NP-011) for treating or preventing ulcerative colitis according to the present invention.

### [Detailed description]

Preferred embodiments of the present invention will be described in detail with reference to the accompanying drawings, but description of well-known technical configurations will be omitted or simplified for ease of description.

### 1. Description of recombinant protein (NP-011) for treating or preventing ulcerative colitis

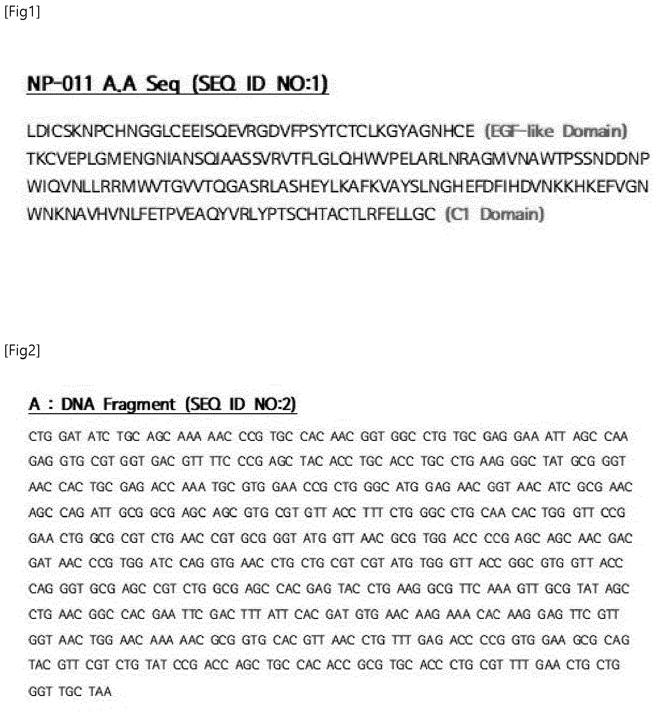

A process of obtaining recombinant protein (NP-011) for treating or preventing ulcerative colitis according to the present invention and structural characteristics of the obtained protein will be described in detail with reference to FIGs. 1 to 5.

### (1) Process of obtaining recombinant protein (NP-011) for treating or preventing ulcerative colitis

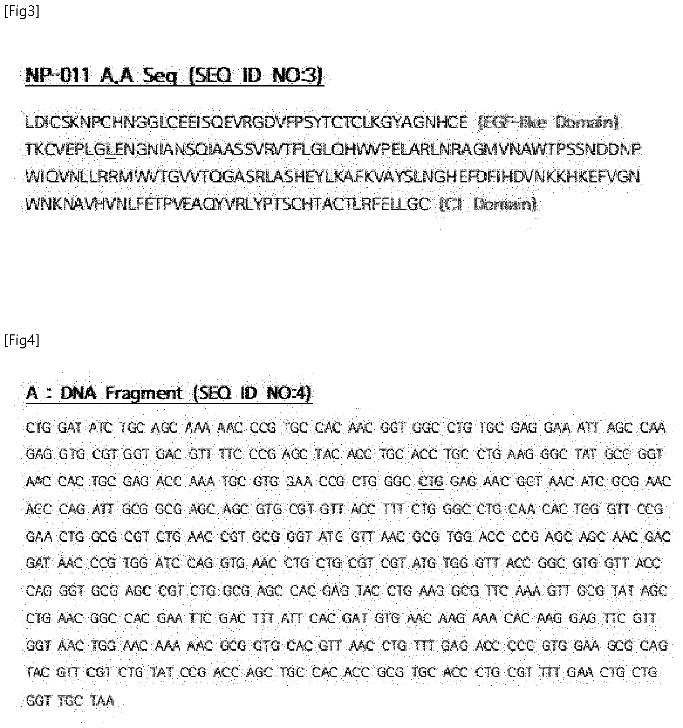

First, an expression process of recombinant protein (NP-011) for treating or preventing ulcerative colitis was performed as follows. In order to improve a structure of milk fat globule-EGF factor 8 (MFG-E8) protein, OriGene's MFG-E8 (NM_005928) Human cDNA Clone (Cat. No. RG217163) was purchased and used as a template, and a PCR process was performed to obtain DNA fragments as shown in FIG. 2 or FIG. 4.

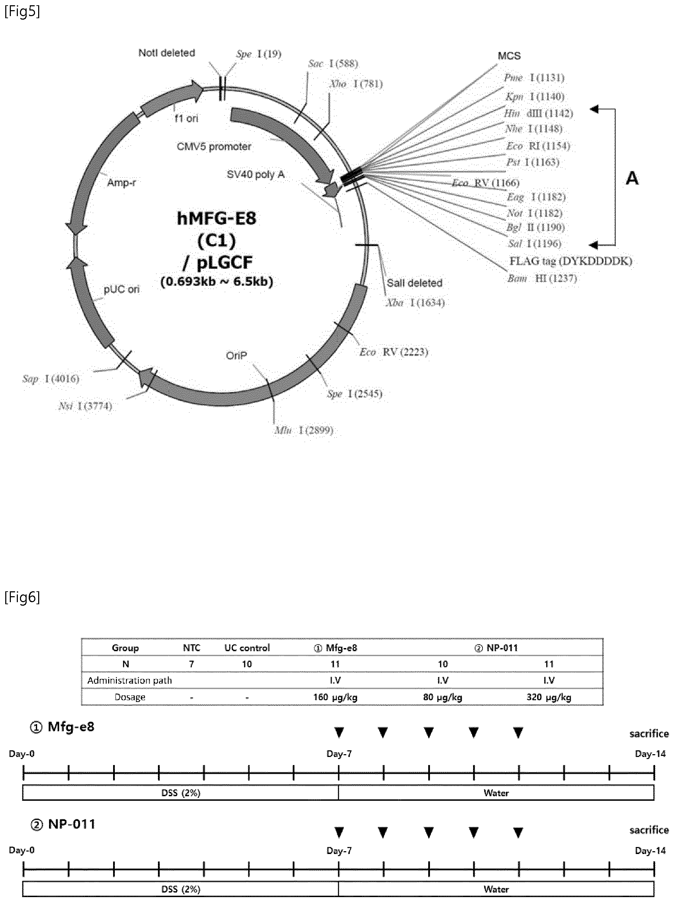

Then, a cloning process of inserting the DNA fragments shown in FIG. 2 or FIG. 4 obtained through the PCR amplification into Hind III and Sal I restriction enzyme sites (site A in FIG. 5) of a pLGCF vector which is a Mammalian expression vector with the structure shown in FIG. 5 was performed.

Then, plasmid DNA was extracted and transfected into HEK293 cells. After 2 days, its culture medium was collected to perform immunoprecipitation (IP) with FLAG resin, and expression of recombinant protein (NP-011) for treating or preventing ulcerative colitis was confirmed using Western blotting. Then, colonies into which an intended NP-011 base sequence (base sequence of SEQ ID NO: 2 or SEQ ID NO: 4 as shown in FIG. 2 or FIG. 4) was inserted were selected to be used for mass production and purification.

Next, a mass production process of the recombinant protein (NP-011) for treating or preventing ulcerative colitis was performed as follows. After securing a large amount of plasmid DNA whose expression has been confirmed through Maxi prep, HEK293 cells were prepared, and then, a large amount of plasmid DNA was transfected into the HEK293 cells to perform mass production of the recombinant protein (NP-011) for treating or preventing ulcerative colitis.

Then, the selected colonies were cultured in a 50 mL flask, the NP-011 expression was verified, and a clone with the highest productivity were selected. A culture solution was primarily recovered by culturing for 7 days in a 5 L fed-batch culture (pH 6.0). A process of primarily removing impure proteins from the recovered culture solution using Q-Sepharose columns and a process of secondarily adsorbing only NP-011 protein using Sp-Sepharose columns were performed to obtain a purity of over 95%, and then, pure NP-011 was obtained by purification based on centrifugation.

In the purification process, in consideration of expression of a FLAG gene at a C-terminal of each protein, only a target protein was bound using FLAG affinity resin, and then, proteins other than the target protein were removed using a washing buffer.

Then, only the pure target protein was extracted using an elution buffer, and then, SDS-PAGE was performed to confirm the finally obtained protein. The production and purity of the target protein was confirmed through Coomassie blue staining and Western blotting using an anti-FLAG antibody.

In the cloning for the expression of the recombinant protein (NP-011) for treating or preventing ulcerative colitis prepared by recombination based on the milk fat globule-EGF factor 8 (MFG-E8) protein, and the mass production and purification thereof, the above-described specific processes are not limiting, and the present invention may be implemented using a variety of techniques that are known to those skilled in the art to construct a specific amino acid sequence structure (SEQ ID NO: 1 (FIG. 1) or SEQ ID NO: 3 (FIG. 3)) of the recombinant protein to be described below.

For example, the structure of the expression vector used for cloning the recombinant protein (NP-011) for treating or preventing ulcerative colitis prepared by recombination based on the milk fat globule-EGF factor 8 (MFG-E8) protein, a transfection target, production conditions and form, and purification conditions and form may be implemented in a variety of ways.

In addition, in relation to the expression of the recombinant protein (NP-011) for treating or preventing ulcerative colitis prepared by recombination based on the milk fat globule-EGF factor 8 (MFG-E8), in a state where the structure of DNA fragments cloned into the expression vector is based on the structure of SEQ ID NO: 2 (FIG. 2) or SEQ ID NO: 4 (FIG. 4), a form in which a specific DNA base sequence that codes a specific signal peptide is additionally connected to the DNA fragments may be used.

### (2) Structure of recombinant protein (NP-011) for treating or preventing ulcerative colitis

The recombinant protein (NP-011) for treating or preventing ulcerative colitis based on the milk fat globule-EGF factor 8 (MFG-E8) protein, prepared through the processes such as cloning, separation, production, and purification of the recombinant protein described above, has an amino acid sequence structure as shown in SEQ ID NO: 1 (FIG. 1) or SEQ ID NO: 3 (FIG. 3) in the following sequence list.

Specifically, the recombinant protein (NP-011) for treating or preventing ulcerative colitis based on the milk fat globule-EGF factor 8 (MFG-E8) protein is recombined into a structure that includes, among EGF-like Domain, C1 Domain, and C2 Domain that represent structural components of the MFG-E8 protein, the EGF-like Domain and the C1 Domain.

In other words, the present invention has such characteristics in which the recombinant protein for treating or preventing ulcerative colitis (NP-011) based on the milk fat globule-EGF factor 8 (MFG-E8) protein has the amino acid sequence of "EGF-like Domain+C1 Domain" as its basic structure and "C2 Domain" is excluded from the structure.

More specifically, the reason why the recombinant protein for treating or preventing ulcerative colitis (NP-011) based on the milk fat globule-EGF factor 8 (MFG-E8) protein has SEQ ID NO: 1 (FIG. 1) or SEQ ID NO: 3 (FIG. 3) in the following sequence list is because the Milk fat globule-EGF factor 8 (MFG-E8) protein has the same Gene ID, but has various versions due to single nucleotide polymorphism (SNP) type mutations.

In fact, NCBI's Human Genome sequence information has been updated to become slightly different from the origin sequence information of the Milk fat globule-EGF factor 8 (MFG-E8) in UniProtBK that is a European protein database. Here, M that is the 53rd amino acid of SEQ ID NO: 1 is modified to L, in which L is a start codon.

In summary, the 53rd amino acid of SEQ ID NO: 3 is modified to L as shown in an underlined part in FIG. 3, and this is because the triplet code corresponding to the 53rd amino acid L of SEQ ID NO: 3 in the DNA base sequence in the structure of DNA fragments has been converted from `ATG' to 'CTG' due to SNP mutation, as shown in an underlined part in FIG. 4.

Accordingly, it is preferable that the recombinant protein for treating or preventing ulcerative colitis (NP-011) based on the milk fat globule-EGF factor 8 (MFG-E8) protein according to the present invention is based on all various versions due to SNP mutation and has the amino acid sequence of "EGF-like Domain+C1 Domain" with "C2 Domain" being excluded in the structure.

The recombinant protein for treating or preventing ulcerative colitis (NP-011) based on the milk fat globule-EGF factor 8 (MFG-E8) protein according to the present invention may be used as a main active ingredient of a pharmaceutical composition used for treating or preventing ulcerative colitis.

In addition, the recombinant protein for treating or preventing ulcerative colitis (NP-011) based on the milk fat globule-EGF factor 8 (MFG-E8) protein according to the present invention and the pharmaceutical composition containing the same as the main active constituent may be formulated for intralesional administration, intravenous administration (artery, vein, or the like), subcutaneous administration, intranasal administration, intraperitoneal administration, or administration in a specific tissue (for example, a lung tissue).

Further, the recombinant protein for treating or preventing ulcerative colitis (NP-011) based on the milk fat globule-EGF factor 8 (MFG-E8) protein according to the present invention may have an additional connection of a specific signal peptide structure in correspondence with a range of structural forms of the DNA fragments cloned into the expression vector in relation to the expression of the recombinant protein described above, as necessary.

For example, the specific signal peptide structure may have an amino acid sequence such as "MPRPRLLAALCGALLCAPSLLVA", and may be connected to the tip of EGF-like Domain.

### 2. Description of results of test for verifying effects of recombinant protein (NP-011) for treating or preventing ulcerative colitis

Regarding the recombinant protein for treating or preventing ulcerative colitis (NP-011) based on the milk fat globule-EGF factor 8 (MFG-E8) protein according to the present invention, a test for verifying effects of treating or preventing ulcerative colitis by the recombinant protein was performed, in which the test employed the following experimental methods for the purpose of defining properties of the recombinant using means that are obvious to those skilled in the art.

### (1) Production and experimental design of ulcerative colitis animal model

First, in the case of an animal model for mice in which ulcerative colitis is induced using DSS (Dextran sulfate sodium), bloody stool, weight loss, large intestinal shrinkage, and mucosal ulcers occur, and histologically, damage occurs in large intestinal epithelial cells, so that the intestinal glands of an epithelial layer fall off without inflammation. Further, the amount of various immune substances in the mucosa increases to cause intestinal inflammation. For this reason, DSS has been widely used to create experimental animal models.

Specifically, in a DSS-induced colitis model in which 9-week-old female C57BL/6 mice were allowed to freely ingest 2% DSS, an experiment was conducted assuming that one C57BL/6 mouse ingested 5 ml of water containing 2% DSS per day.

For 7 days, only water containing 2% DSS without any treatment was provided to the mice, and for 5 days after the 7th day, water without 2% DSS was provided, and simultaneously, various comparative substances, including NP-011 protein, were fed at various concentrations by injection into the caudal vein, thereby creating an animal model of inducing ulcerative colitis.

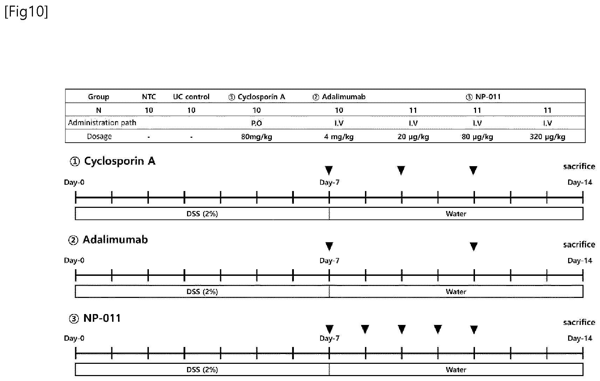

As shown in FIGs. 6 and 10, an `NTC' control group that is a control group was continuously supplied with only water from day 0, and a `UC CONTROL' control group that is a positive control group was supplied with only water containing 2% DSS from day 0 to day 7, and then supplied with plain water from day 7 to day 14.

In addition, as shown in FIG. 6, an 'MFG-E8' experimental group that is an experimental group was supplied with only water containing 2% DSS from day 0 to day 7, was supplied with plain water from days 7 to 14, and MFG-E8 protein of 160 µg/kg was administered once a day for 5 days starting from day 7, a total of 5 times.

Then, as shown in FIG. 10, a `Cyclosporin A' experimental group that is an experimental group was supplied with only water containing 2% DSS from day 0 to day 7, was supplied with plain water from day 7 to day 14, and a Cyclosporin A ingredient of 80ug/kg was administered once a day at 2-day intervals starting from day 7, a total of 3 times.

In addition, as shown in FIG. 10, an `Adalimumab' experimental group that is an experimental group was supplied with only water containing 2% DSS from day 0 to day 7, was supplied with plain water from day 7 to day 14, and an Adalimumab ingredient of 80 mg/kg was administered once daily on day 7 and day 11, a total of 2 times.

Next, as shown in FIG. 10, a first experimental group 'NP-011' that is an experimental group was supplied with only water containing 2% DSS from day 0 to day 7, was supplied with plain water from day 7 to day 14, and NP-011 recombinant protein of 20 µg/kg was administered once a day for 5 days starting from day 7, a total of 5 times.

In addition, as shown in FIGs. 6 and 10, a second experimental group 'NP-011' that is an experimental group was supplied with only water containing 2% DSS from day 0 to day 7, was supplied with plain water from day 7 to day 14, and NP-011 recombinant protein of 80 µg/kg was administered once a day for 5 days starting from day 7, a total of 5 times.

Lastly, as shown in FIG. 6 and FIG. 10, a third experimental group 'NP-011' that is an experimental group was supplied with only water containing 2% DSS from day 0 to day 7, was supplied with plain water from day 7 to day 14, and NP-011 recombinant protein of 320 µg/kg was administered once a day for 5 days starting from day 7, a total of 5 times.

The following experiments using all animal models created in this way were conducted under approval of the Institutional Animal Care and Use Committee of NEXEL Co., Ltd., while following animal care regulations.

### (2) Verification of symptom relief in ulcerative colitis animal model - related to damage to mucosa of large intestine

First, using the animal models corresponding to the `NTC' control group, the `UC CONTROL' control group, the `MFG-E8' experimental group, the 'NP-011' second experimental group, and the 'NP-011' third experimental group, for each animal model group, the cecum was removed from a large intestinal tissue, and 1 to 2 cm of the proximal and 1 cm of the anus were secured and fixed with 4% paraformaldehyde.

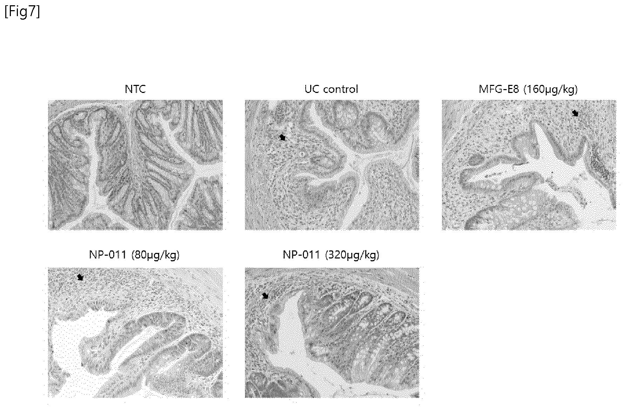

Then, the tissue fixed with 4% paraformaldehyde was subjected to staining (Hematoxylin & Eosin stain) and histopathological examination by KP&T Co., Ltd.. The pathological interpretation was made with reference to INHAND (International Harmonization of Nomenclature and Diagnostic Criteria for Lesions) which is an international standard toxicology glossary. The degrees of crypt damage, inflammation severity, and inflammation extent in the mucosa of the large intestinal tissue were measured for comparison in the respective animal model groups, and results thereof are shown in FIG. 7 and Table 1. (Reference-J Toxicol Pathol 2016; 29 (1 Suppl): 1S-124S)

**[Table 1]**

| Group | NTC | UC | MGF-E8 | NP-011 | |
|---|---|---|---|---|---|
| Dosage | | | 160µg/kg | 80µg/kg | 320µg/kg |
| Number of Animals | 7 | 10 | 11 | 10 | 11 |
| Inflammation Severity | 0.0 | 1.2 | 1.1 | 0.7 | 0.8 |
| Inflammation Extent | 0.0 | 1.4 | 1.2 | 0.6 | 0.7 |
| Crypt Damage | 0.0 | 4.7 | 2.9 | 3.7 | 3.0 |
| Group total average | 0.0 | 7.3 | 5.2 | 4.9 | 4.5 |

Form the results in FIG. 7 and Table 1, it can be confirmed that the degrees of crypt damage, inflammation severity, and inflammation extent in the mucosa were reduced in the order of MFG-E8 160 gg/kg, NP-011 80 gg/kg, and NP-011 320 gg/kg compared with the positive control group after induction of ulcerative colitis, so that the overall degree of damage to the large intestinal mucosa was lowered.

Next, using the animal models corresponding to the `NTC' control group, the `UC CONTROL' control group, the `Cyclosporin A' experimental group, the 'Adalimumab' experimental group, the 'NP-011' first experimental group, the 'NP-011' second experimental group, and the 'NP-011' third experimental group, the cecum was removed from the large intestinal tissue for each animal model group, and 1 to 2 cm of the proximal and 1 cm of the anus were secured and fixed with 4% paraformaldehyde.

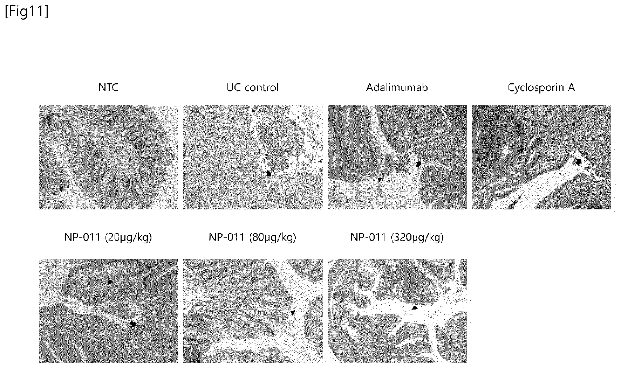

Then, the tissue fixed with 4% paraformaldehyde was subjected to staining (Hematoxylin & Eosin stain) and histopathological examination by KP&T Co., Ltd.. The pathological interpretation was made with reference to INHAND. The degrees of erosion and epithelial hyperplasia in the mucosa of the large intestinal tissue were measured for comparison of lesions in the respective animal model groups, and results thereof are shown in FIG. 11 and Table 2. (Reference-J Toxicol Pathol 2016; 29 (1 Suppl)): 1S-124S)

**[Table 2]**

| Group | NTC | UC | AD | CYA | NP-011 | | |
|---|---|---|---|---|---|---|---|
| Dosage | | | 4mg/kg | 80mg/kg | 20µg/kg | 80µg/kg | 320µg/kg |
| Number of Animals | 9 | 10 | 10 | 10 | 11 | 11 | 11 |
| Erosion, mucosa | 0.0 | 2.2 | 0.8 | 1.5 | 1.1 | 1.3 | 0.5 |
| Epidermal Hyperplasia | 0.0 | 4.4 | 4.2 | 4.3 | 4.8 | 3.4 | 3.5 |
| Group total average | 0.0 | 6.6 | 5.0 | 5.8 | 5.9 | 4.7 | 4.0 |

From the results in FIG. 11 and Table 2, it can be confirmed that the degrees of erosion and epidermal hyperplasia in the mucosa of large intestinal tissues were reduced in Cyclosporin A and NP-011 20 µg/kg to the same extent as in the positive control group after induction of ulcerative colitis, and were reduced in the order of Adalimumab, NP-011 80 µg/kg, and NP-011 to 320 µg/kg, so that the overall degree of damage to the large intestinal mucosa was lowered.

### (3) Verification of symptom relief in ulcerative colitis animal model - related to body weight change

First, using the animal models corresponding to the `NTC' control group, the `UC CONTROL' control group, the `MFG-E8' experimental group, the 'NP-011' second experimental group, and the 'NP-011' third experimental group, body weight was measured daily for each animal model group before drug administration to reduce errors due to the administration.

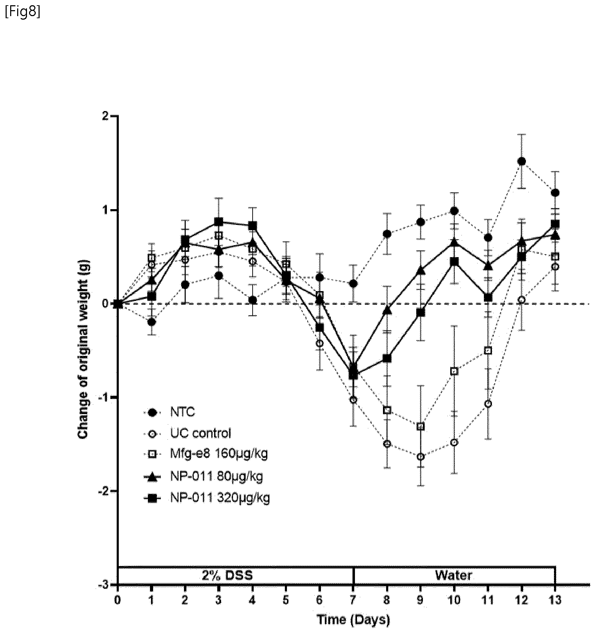

In addition, increase or decrease in body weight was analyzed compared with the positive control group, and comprehensive results thereof are shown in FIG. 8 and Table 3.

**[Table 3]**

| Dunnett's multiple comparisons test | | UC VS NTC | UC VS MFG-E8 | UC VS NP-011 (80µg/kg) | UC VS NP-011 (320µg/kg) |
|---|---|---|---|---|---|
| Day 7 | Summary | ** | ns | ns | ns |
| | P Value | 0.0042 | 0.6775 | 0.6849 | 0.8575 |
| Day 8 | Summary | **** | ns | **** | * |
| | P Value | <0.0001 | 0.6634 | <0.0001 | 0.0241 |
| Day 9 | Summary | **** | ns | **** | **** |
| | P Value | <0.0001 | 0.7402 | <0.0001 | <0.0001 |
| Day 10 | Summary | **** | ns | **** | **** |
| | P Value | <0.0001 | 0.0786 | <0.0001 | <0.0001 |
| Day 11 | Summary | **** | ns | **** | ** |
| | P Value | <0.0001 | 0.2654 | <0.0001 | 0.0029 |

From the test results in FIG. 8 and Table 3, it can be confirmed that there was a statistically significant decrease in weight in the NP-011 second and third experimental groups from day 8 compared with the positive control group (UC) after induction of ulcerative colitis (****P < 0.0001, ***P < 0.001, **P < 0.01, *P < 0.05).

Next, using the animal models corresponding to the `NTC' control group, the `UC CONTROL' control group, the `Cyclosporin A' experimental group, the 'Adalimumab' experimental group, the 'NP-011' first experimental group, the 'NP-011' second experimental group, and the 'NP-011' third experimental group, body weight was measured daily for each animal model group before drug administration to reduce errors due to the administration.

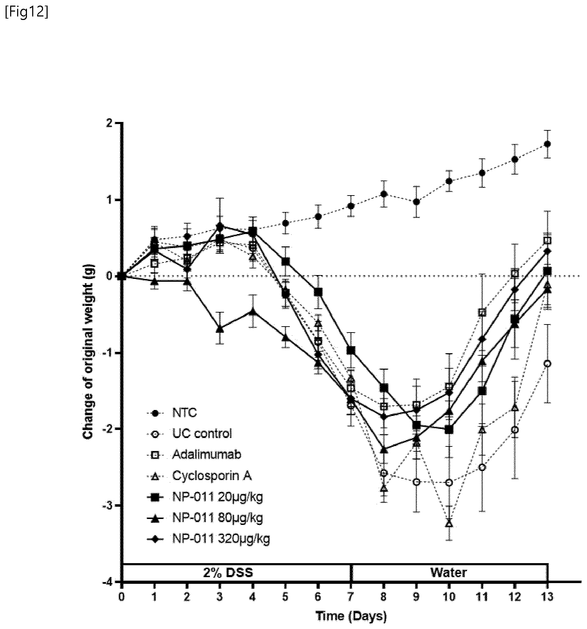

In addition, increase or decrease in body weight was analyzed on the basis of the original body weight, and the level of weight increase or decrease compared with the positive control group was also analyzed. Comprehensive results thereof are shown in FIG. 12, Table 4, and Table 5.

**[Table 4]**

| Change of original weight (g) | NTC | UC | AD | CYA | NP-011 (20µg/kg) | NP-011 (80µg/kg) | NP-011 (320µg/kg) |
|---|---|---|---|---|---|---|---|
| Day 1 | 0.478± 0.175 | 0.452± 0.159 | 0.161± 0.124 | 0.439± 0.189 | 0.360± 0.145 | -0.062± 0.107 | 0.336± 0.112 |
| Day 2 | 0.522± 0.169 | 0.380± 0.146 | 0.237± 0.098 | 0.202± 0.206 | 0.398± 0.223 | -0.062± 0.131 | 0.091± 0.197 |
| Day 3 | 0.628± 0.157 | 0.474± 0.153 | 0.438± 0.108 | 0.493± 0.147 | 0.485± 0.188 | -0.678± 0.205 | 0.661± 0.361 |
| Day 4 | 0.609± 0.168 | 0.367± 0.170 | 0.411± 0.112 | 0.260± 0.154 | 0.591± 0.190 | -0.456± 0.221 | 0.544± 0.185 |
| Day 5 | 0.695± 0.141 | -0.246± 0.174 | -0.217± 0.178 | -0.174± 0.112 | 0.191± 0.191 | -0.793± 0.137 | -0.240± 0.158 |
| Day 6 | 0.780± 0.152 | -0.859± 0.202 | -0.846± 0.258 | -0.609± 0.101 | -0.208± 0.218 | -1.129± 0.149 | -1.025± 0.186 |
| Day 7 | 0.921± 0.175 | -1.691± 0.269 | -1.464± 0.242 | -1.330± 0.095 | -0.965± 0.233 | -1.606± 0.203 | -1.600± 0.210 |
| Day 8 | 1.076± 0.173 | -2.574± 0.300 | -1.702± 0.283 | -2.764± 0.191 | -1.459± 0.246 | -2.261± 0.188 | -1.839± 0.236 |
| Day 9 | 0.974± 0.139 | -2.689± 0.397 | -1.683± 0.338 | -2.179± 0.215 | -1.948± 0.248 | -2.109± 0.285 | -1.752± 0.313 |
| Day 10 | 1.245± 0.136 | -2.697± 0.473 | -1.444± 0.443 | -3.230± 0.224 | -2.003± 0.371 | -1.756± 0.303 | -1.522± 0.318 |
| Day 11 | 1.352± 0.186 | -2.500± 0.574 | -0.471± 0.500 | -2.006± 0.306 | -1.500± 0.468 | -1.106± 0.274 | -0.820± 0.352 |
| Day 12 | 1.528± 0.192 | -2.010± 0.636 | -0.035± 0.385 | -1.715± 0.397 | -0.556± 0.527 | -0.620± 0.310 | -0.177± 0.275 |
| Day 13 | 1.728± | -1.143± | -0.467± | -0.109± | -0.067± | -0.166± | 0.328± |
| | 0.178 | 0.513 | 0.387 | 0.264 | 0.479 | 0.270 | 0.238 |

**[Table 5]**

| Dunnett's multiple comparisons test | | UC VS NTC | UC VS AD | UC VS CYA | UC VS NP-011 (20µg/kg) | UC VS NP-011 (80µg/kg) | UC VS NP-011 (320µg/kg) |
|---|---|---|---|---|---|---|---|
| 10 day s | Summary | **** | ** | ns | ns | * | ** |
| | P Value | <0.0001 | 0.0047 | 0.515 | 0.2331 | 0.0497 | 0.0074 |
| Day 11 | Summary | **** | **** | ns | * | *** | **** |
| | P Value | <0.0001 | <0.0001 | 0.591 | 0.032 | 0.0009 | <0.0001 |
| Day 12 | Summary | **** | **** | ns | *** | *** | **** |
| | P Value | <0.0001 | <0.0001 | 0.9269 | 0.0005 | 0.0009 | <0.0001 |
| Day 13 | Summary | **** | **** | * | ** | * | *** |
| | P Value | <0.0001 | <0.0001 | 0.0295 | 0.0053 | 0.0382 | 0.0004 |

From the results in FIG. 12, Table 4, and Table 5, it can be confirmed that weight loss was observed in all of the positive control group (UC control), the Adalimumab group, the Cyclosporine A group, and the NP-011 groups (20 to 80 µg/kg), and a suppression effect of weight loss induced from colitis was shown in the NP-011 (80 µg/kg) and the Cyclosporine A to the same extent as in the positive control group, and was shown in the order of the NP-011 group (20 pg/kg), the NP-011 group (320 pg/kg), and the Adalimumab group (****P < 0.0001, ***P < 0.001, **P < 0.01, *P < 0.05).

### (4) Verification of symptom relief in ulcerative colitis animal model - related to colon length change

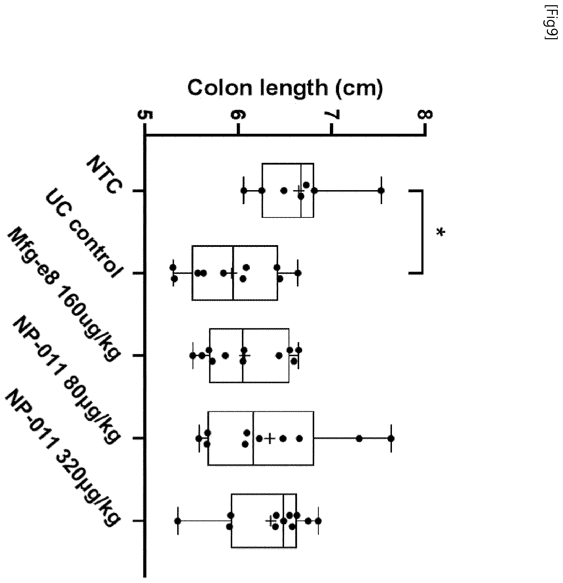

First, using the animal models corresponding to the `NTC' control group, the `UC CONTROL' control group, the `MFG-E8' experimental group, the 'NP-011' second experimental group, and the 'NP-011' third experimental group, the large intestine was extracted separately for each animal model group, the colon length was analyzed, and the results thereof are shown in FIG. 9.

Specifically, to analyze the colon length, each animal model was euthanized by gradual increase in carbon dioxide 3 days after the last administration, and the large intestine was extracted. The extracted large intestine was photographed with a length measuring device (30 cm ruler), and the obtained photograph was quantified using image J.

From the result in FIG. 9, it can be confirmed that there was a statistically significant increase in the colon length shrinkage suppression effect in the MFG-E8 group and the NP-011 groups (80 and 320 ug/kg) compared with the non-administration control group after induction of ulcerative colitis, and the 'NP-011' second experimental group and the 'NP-011' third experimental group were superior to the `MFG-E8' experimental group in the colon length shrinkage suppression effect.

Here, a black cross in the center of each box shown in FIG. 9 means an average value, and the box shows the distribution of values in each group ranging from 25% to 75% (**** P < 0.0001, *** P < 0.001, ** P < 0.01, * P < 0.05).

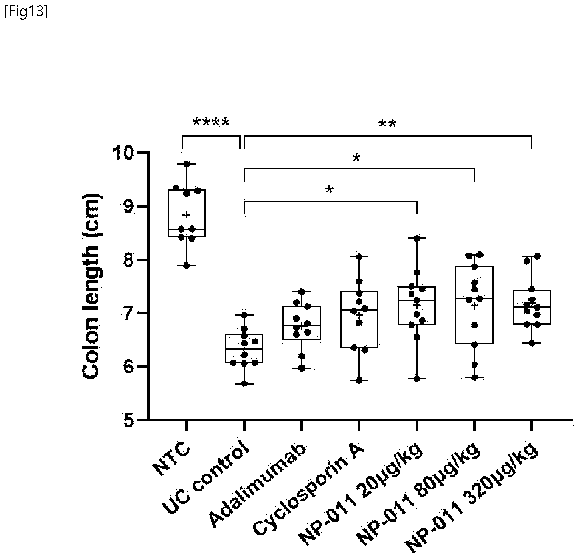

Next, using the animal models corresponding to the `NTC' control group, the `UC CONTROL' control group, the `Cyclosporin A' experimental group, the 'Adalimumab' experimental group, the 'NP-011' first experimental group, the 'NP-011' second experimental group, and the 'NP-011' third experimental group, the large intestine was extracted for each animal model group, the colon length was analyzed, and results thereof are shown in FIG. 13.

Specifically, to analyze the colon length, each animal model was euthanized by gradual increase in carbon dioxide 3 days after the last administration, and the large intestine was extracted. The extracted large intestine was photographed with a length measuring device (30 cm ruler), and the obtained photograph was quantified using image J.

From the result in FIG. 13, it can be confirmed that the colon length increased to a statistically significant degree in the respective NP-011 groups (20, 80, and 320 ug/kg) compared with the positive control group after induction of ulcerative colitis.

Here, a black cross in the center of each box shown in FIG. 13 means an average value, and the box shows the distribution of values in each group ranging from 25% to 75% (**** P < 0.0001, *** P < 0.001, ** P < 0.01, * P < 0.05).

### (5) Verification of symptom relief in ulcerative colitis animal model - related to IL-6 concentration in blood

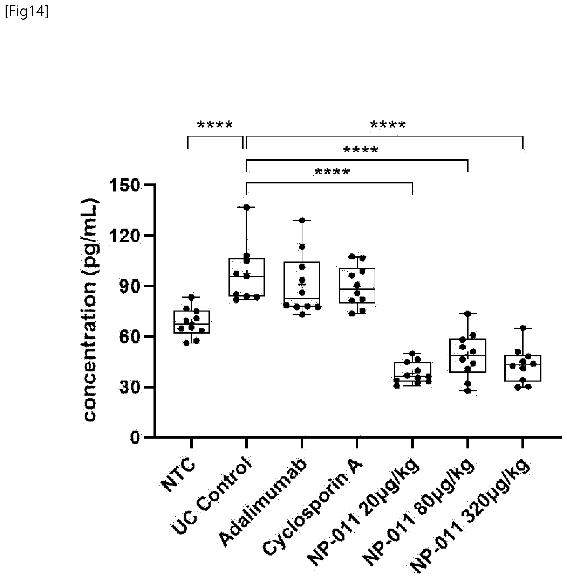

First, using the animal model corresponding to the `NTC' control group, the `UC CONTROL' control group, the `Cyclosporin A' experimental group, the 'Adalimumab' experimental group, the 'NP-011' first experimental group, the 'NP-011' second experimental group, and the 'NP-011' third experimental group, blood collected through a heparinized capillary tube for each animal model group was stored on ice for 30 minutes, was centrifuged at 12,000 rpm at 4°C for 15 minutes to separate serum excluding blood clots, and the result was stored at -80°C. Then, for analysis, the stored serum was slowly melted at 4°C, and was diluted with PBS (1% BSA). Then, the concentration of IL-6 in blood was measured using an ELISA kit, and results thereof are shown in FIG. 14.

Here, interleukin-6 (IL-6) is a pro-inflammatory cytokine widely identified in diagnosing inflammatory diseases, including TNF-α and IL-1β, and is one of cytokines that send signals through the JAK/STAT pathway associated with various inflammatory diseases. The interleukin-6 (IL-6) is necessary for differentiating naive T cells into Th17 cells which may cause inflammation.

Accordingly, the 'NP-011' first experimental group, the 'NP-011' second experimental group, and the 'NP-011' third experimental group showed a lower blood concentration of IL-6, compared with the `UC CONTROL' control group, which shows suppression of inflammation aggravation.

In summary, it can be confirmed from FIG. 14 that the 'NP-011' first experimental group, the 'NP-011' second experimental group, and the 'NP-011' third experimental group showed statistically significant reduction of a concentration of IL-6 in blood compared with the `UC CONTROL' control group.

Here, a black cross in the center of each box shown in FIG. 14 means an average value, and the box shows the distribution of values in each group ranging from 25% to 75% (**** P < 0.0001, *** P < 0.001, ** P < 0.01, * P < 0.05).

The examples disclosed in the present invention are not intended to limit but illustrate the technical idea of the present invention, and the scope of the technical idea of the present invention is not limited by these examples. The scope of protection should be interpreted in accordance with the claims below, and all technical ideas within the equivalent scope should be interpreted as being included in the scope of ∘ the present invention.

## Claims

1. Recombinant protein for ulcerative colitis treatment or prevention that is recombinant protein based on milk fat globule-EGF factor 8 (MFG-E8) protein, consisting of an amino acid sequence of SEQ ID NO: 1 or SEQ ID NO: 3.

2. The recombinant protein according to claim 1, wherein the recombinant protein reduces, compared with a case in which the recombinant protein is not administered, degrees of crypt damage, an inflammation severity, and an inflammation extent in a mucosa of a large intestinal tissue that is a target for treatment or prevention.

3. The recombinant protein according to claim 1, wherein the recombinant protein reduces, compared with a case in which the recombinant protein is not administered, degrees of erosion and epithelial hyperplasia in a mucosa of a large intestinal tissue that is a target for treatment or prevention.

4. The recombinant protein according to claim 1, wherein the recombinant protein reduces, compared with a case in which the recombinant protein is not administered, a concentration of interleukin-6 (IL-6) in blood of a target for treatment or prevention.

5. The recombinant protein according to claim 1, wherein the recombinant protein reduces, compared with a case in which the recombinant protein is not administered, degrees of weight loss and colon length shrinkage caused by ulcerative colitis of a target for treatment or prevention.

6. The recombinant protein according to claim 1, wherein the recombinant protein is formulated for intralesional administration, intravascular administration, subcutaneous administration, intranasal administration, or intraperitoneal administration.

7. A composition for ulcerative colitis treatment or prevention containing the recombinant protein according to any one of claims 1 to 6 as an active ingredient.

8. A gene encoding the recombinant protein according to any one of claims 1 to 6.

9. A recombinant vector comprising a gene encoding the recombinant protein according to any one of claims 1 to 6.
